# EUROPEAN PATENT SPECIFICATION

(11) **EP 2 380 549 B1**
(45) Date of publication and mention of the grant of the patent: **06.03.2013**
(21) Application number: 10425125.1
(22) Date of filing: 21.04.2010
(51) Int. Cl.: A61J 7/00, B65B 57/20, B65G 1/137

(54) **Counter for dispensing drugs in form of tablets in personal blisters**
Zähler zur Abgabe von Medikamenten in Form von Tabletten in persönlichen Blisterpackungen
Compteur pour la distribution de médicaments sous forme de comprimés en plaquettes thermoformées personnels

(43) Date of publication of application: 26.10.2011
(73) Proprietor: Frate, Giovanni, 35123 Padova (IT)
(72) Inventor: Frate, Giovanni, 35123 Padova (IT)
(74) Representative: Gallo, Luca

(56) References cited:
- EP-A1- 0 208 029
- US-A1- 2001 019 065
- US-A1- 2007 084 150

## Description

### DESCRIPTION

The present invention refers to a counter for dispensing drugs in form of tablets in personal blisters for patients according to the respective medical prescriptions.

The counter in question is intended to be used in hospitals and in particular in retirement homes with the aim of administering - thorough special container personal blisters -the daily dose of drugs to the patients.

Therefore, the present invention refers to the industrial sector of manufacturing repackaging machines as well as the health services industry and retirement homes.

As known, distribution of drugs for example in a retirement home requires that a personal blister containing the various tablets administered thereto during the day be prepared daily for each patient.

Obviously, preparation of the personal blister must comply with the medical prescription and may require manipulating numerous packages of drugs, for completion thereof. Furthermore, in a retirement home hosting several patients the operators are required to manage tens or hundreds of personal blisters.

Errors regarding administration of drugs may lead to extremely serious consequences for the patients. The errors may retard the interpretation of the medical prescriptions, preparation of the personal blisters, administration to patients i.e. the correct association of the personal blisters and thus the drugs of the patients.

Further important problems regarding administration of drugs regard the control of the use expiry dates, declared by the manufacturer and thus use if prohibited beyond a given date, management of the stocked drugs, monitoring the drugs distribution activity from medical prescription to preparation of personal blisters up to administration to patients. Monitoring is required both for statistic and diagnostic purposes on all aspects related to verifying the result of administering the drug to the patient, both with the aim of correctly identifying and attributing responsibilities due to erroneous distribution of drugs.

Currently, as known, once the doctor writes the prescription for each patient, the operator is required to prepare - daily - personal blisters taking the drugs from the cabinets and placing them in the amounts prescribed in the single compartments into which the personal blister is divided for administering drugs at different times of the day.

The patient's data is usually indicated by means of a label on the personal blister, in such a manner that once prepared, the latter may be correctly delivered to the patient usually by another operator who will also see to administering the drugs.

In practice, this administration process has revealed serious drawbacks.

A first drawback lies in the fact that there is no certainty whether the first operator shall correctly interpret the medical prescription, which - as known - is usually written with an indistinguishable handwriting. Furthermore and above all there is no certainty that the same operator inserts the various types of drugs into the personal blisters associating them to the patient correctly, just like there is no assurance to meeting the prescribed posology or administration time separating the drugs correctly in the specific compartments of the personal blister.

Given that there is usually no control or monitoring of the activities provided for, it is not even possible to attribute - in case of an error - individual responsibilities and identify remedies to the error based on accurate deduction.

A second drawback, maybe even more serious than the previous one, lies in the fact that an operator may erroneously administer drugs of personal blisters to patients different from those the personal blister was intended for. As a matter of fact, currently there are no means for preventing such event and hence, especially if the operator does not know the patients by name i.e. in extensive facilities there are high chances of administering drugs to the wrong patients.

A third drawback observable in processes for distributing drugs used up to date in retirement homes regards the method for storing the drugs in the cabinets and controlling the respective expiry dates.

The stocked products are currently managed - as regards with most drugs - referring to the single patient providing enough stock with the aim of avoiding the risk of running out of the drug.

This method for storing drugs requires storing a large amount of drugs in generally cumbersome cabinets and implies engaging a considerable amount of time for the personnel to check the actual expiry date and avoid using expired drugs.

Retirement homes currently store considerable amounts of drugs and the time required to check the respective expiry dates has a negative impact on the performance of the entire service thus implying considerable costs. Health services literature reveals more and more reference to errors regarding distribution of drugs with extremely serious imaginable effects. In addition, in most cases there actually is no systematic monitoring of all activities related to the use of drugs that may lead to technical analysis of the results and identifying erroneous conduct and responsibilities.

Therefore, in retirement homes, the current quality of the service of distributing drugs is solely entrusted onto the ability and seriousness of the operators to work in a carful and disciplined manner but without any actual control on the actions thereof.

Patent EP 208029 describes an apparatus for dispensing drugs in form of pills which solely partly overcomes the abovementioned drawbacks. The patent in question provides for arranging compartments mounted on each other, wherein the tablets to be dispensed are contained in cardboard strips accommodated in grooves. Provided for is a unit for distributing drugs mounted on a carriage, controlled by a computer and moveable between the compartments due to the use of racks. Suitable means for gripping the distribution unit allow picking the tablets from the strips, then the tablets are dropped onto a conveyor belt to be moved up to a tray. The latter is then moved towards a control station where an operator may check - on the monitor - the correctness of the product picked previously. This apparatus does not allow manually controlling the single drug and costs thereof are definitely high and it requires supplying the drugs in very particular and special packages i.e. in form of strips of tablets, thus leading to the pharmaceutical producers sharing the costs.

Available in the market are various solutions for apparatus that provide for the storage of drugs in containers and thus suitable means for picking the tablets from the abovementioned containers for example by means of hopper augers, tablet counters and other means.

Such apparatus substantially allow distributing the tablets starting from a storage element already stocked and made up of containers of a high number of tablets. This storage method is not allowed by many national health systems which require that the tablets be contained in sealed packaging blisters until use thereof. Thus, they require means for moving and picking tablets that are quite complex and financially expensive. On the other hand, the number of types of drugs is materially quite low.

In addition these systems do not offer sufficient guarantees that the drugs are subsequently administered correctly to the patient through the personal blister intended therefor.

In this context, the main aim of the present invention is thus that of overcoming the drawbacks revealed by the apparatus of the known type, by providing a counter for dispensing drugs in personal blisters that is particularly safe offering the patients the highest guarantee that the prescribed drugs are administered correctly to the patients.

Further object of the present invention is that of providing a counter for dispensing drugs in form of tablets in personal blisters that does not limit the number of type of drugs. Further object of the present invention is that of providing a counter for dispensing drugs in form of tablets in personal blisters that is also capable of eliminating or reducing the time for checking and finding the drugs about to expire to the maximum.

Further object of the present invention is that of providing a counter for dispensing drugs in form of tablets in personal blisters capable of allowing monitoring the entire administration activity to reduce chances of errors and correctly attribute responsibility should such errors occur.

Further object of the present invention is that of providing a counter for dispensing drugs in form of tablets in personal blisters that is simple and operatively entirely reliable.

The technical characteristics of the invention, according to the abovementioned objects, are clearly observable from the contents of the claims indicated below and the advantages of the same shall be clearer in the detailed description that follows, provided referring to the attached drawings and photos, which represent an embodiment thereof strictly for exemplifying and non-limiting purposes wherein:
- figure 1 shows an overall perspective of the counter for dispensing drugs in form of tablets in personal blisters subject of the present invention in an exemplifying and non-limiting view;

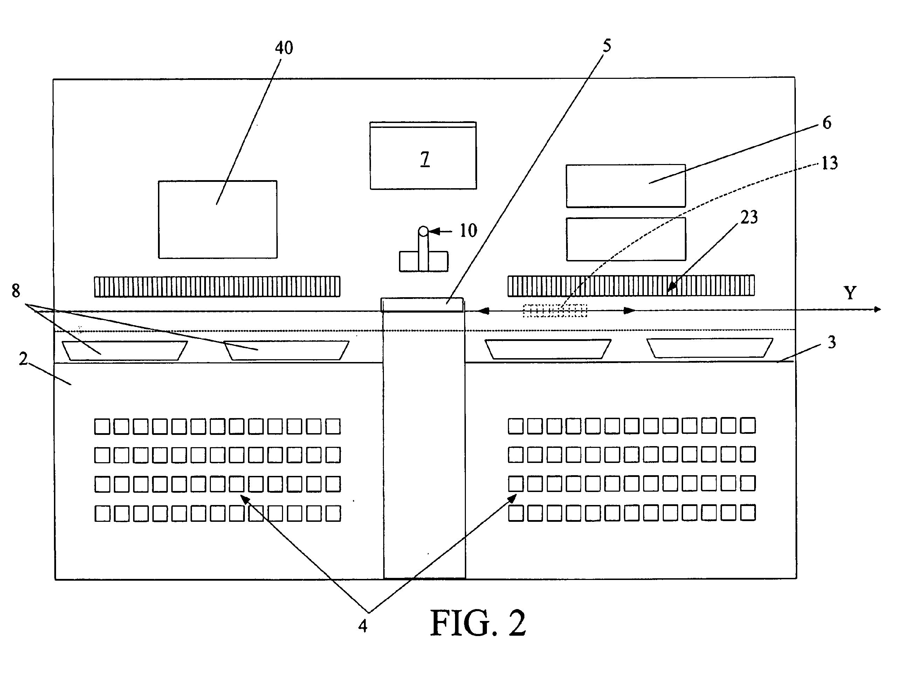

- figure 2 schematically shows, in a front view, the counter of figure 1;

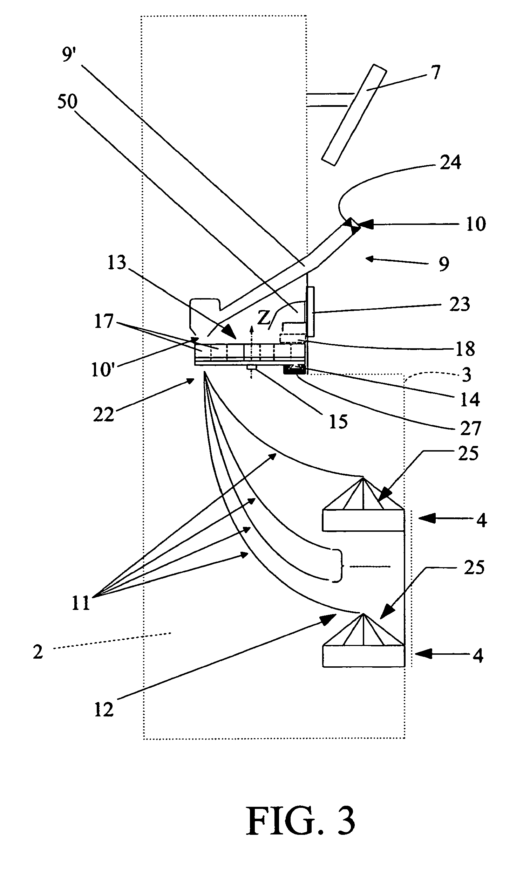

- figure 3 schematically shows, in a side view, the counter of figure 1, with some parts removed to show other parts better;

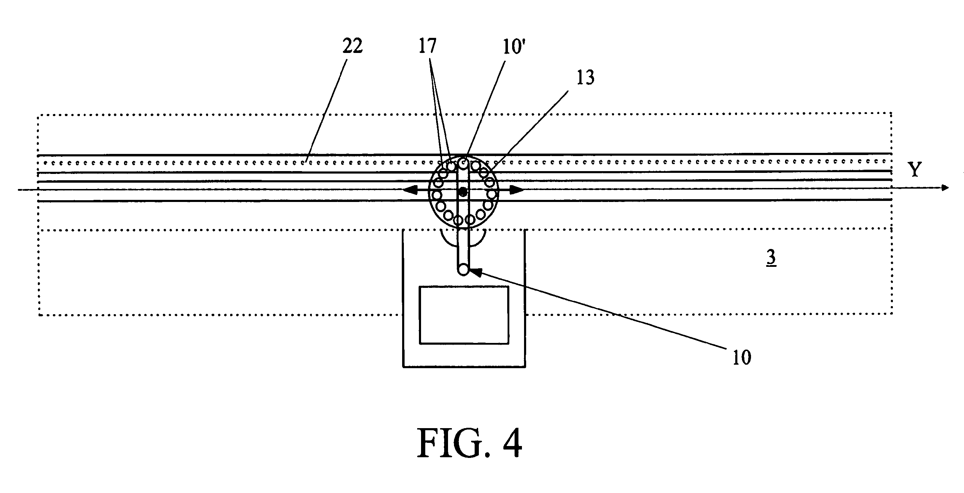

- figure 4 schematically shows, in a plan view, the counter for dispensing drugs in form of tablets in personal blisters of figure 1;
- figure 5 schematically shows a detail of the counter subject of the invention, regarding a distributor unit positioned over the cell of a personal blister;

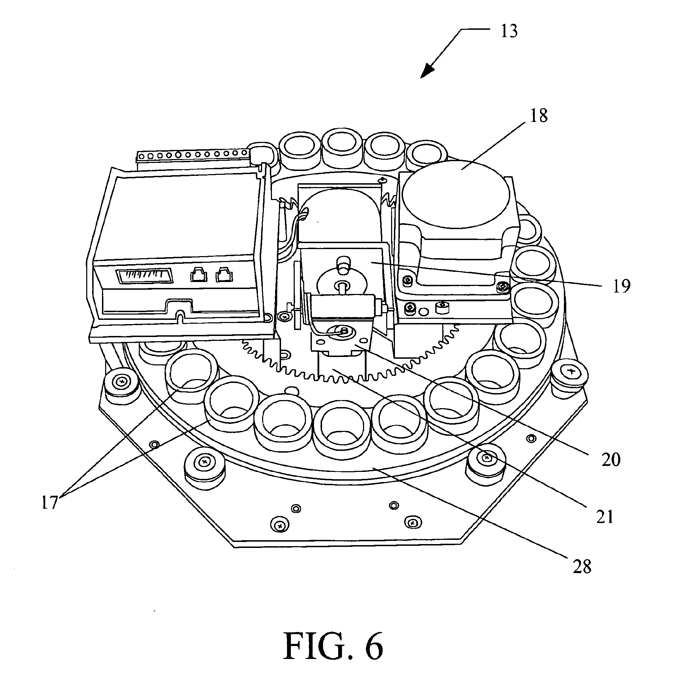

- figure 6 schematically shows a detail of the counter subject of the invention, regarding an intermediate storage element.

According to the figures of the attached drawings, the counter for dispensing drugs subject of the present invention is indicated in its entirety with 1.

Such counter comprises a bearing framework 2 lying against the ground, provided with a central operating station in which an operator may stay seated or on his feet for operations of loading the personal blisters as described hereinafter.

As known, the term personal blister is commonly used to indicate a container, used for holding pharmaceutical products, obtained through a process thermoforming a film made of PVC or other resin material and provided with some compartments for holding the drugs to be administered at various times of the day (typically 4 compartments referring to morning, midday, afternoon and evening).

Provided for is a support surface 3 extended with two sections to the right and to the left with respect to the operator. Positioned on such surface 3 may be plastic containers or drawers 8 for accommodating, for example on the right, packages of drugs yet to be used and on the left those already used though still containing drugs therein.

Positioned on the ground may be a special basket for collecting packages of used drugs. Arranged adjacent to each other on several levels beneath the support surface 3 and over the entire extension of the bearing framework 2 are container cells 4, each of which is uniquely associable to a single personal blister of a patient through a mechanical, electrical or electronic key. The personal blister is inserted into the cell for loading, into the various compartments, with the drugs indicated in the therapy record of the patient, after adding the tablets, it is extracted from the cell and then taken to the patient for administration.

A visual or sound signal triggered for example by an electrical contact allows signalling failed insertion of a personal blister or an erroneous insertion. Preferably, the presence of luminous leds shall indicate that the personal blister is at an erroneous position or not fitted properly.

Each personal blister is in other words strictly personal, bears the patient's data, an identification number thereof and possibly an RFi (Radio Frequency identification) or a barcode and may be fitted solely into its intended compartment.

The processor allows starting the process of dispensing the drugs strictly solely when the personal blisters are positioned into the respective cells.

The counter 1 is provided with a recognition unit 5 capable of reading the barcode borne on the packages of the drugs to identify the type thereof.

Such unit 5 may be made up of a reader in form of an optical pen or scanner or it may be recessed inside the support surface protected behind a horizontal transparent surface on which the packages may be placed for identification thereof.

Furthermore, provided for directly on the support structure 2 or simply connected remotely is an computer 6 provided with a monitor 7 preferably arranged in front of the operating station.

The computer is in communication with the recognition unit 5 and it is provided with a data base stored on which are: the information related to the drugs per active ingredient, commercial name, and packaging with indications regarding the expiry date and the number of tablets contained therein; information regarding the guests with the respective therapy record constantly updated by the doctor in charge.

According to a preferred solution of the present invention, the data base also contains an electronic store for all drugs stocked in the retirement home. For such purpose, all drug packages that are stored electronically are loaded through the recognition unit 5. This - as observable hereinafter - allows monitoring the storage element status in real time in such a manner to be able to check - at any time - the available stock. Indicated hereinafter, after describing the process for dispensing the drugs, are all operating options that may be identified by the computer.

Operatively, the computer allows, during the step of distributing (or unloading) the tablets, displaying - for each drug identified by the recognition unit 5 - the overall number of tablets to be supplied to the patients to whom it is prescribed alongside the list of patients for whom the same drug is prescribed.

At a position easily reachable from the operating station it is possible to gain access to an introduction element 9 provided with an opening 10 into which the operator introduces - singularly - the tablets of the identified drug up to the number displayed on the monitor 7. It is solely after all the tablets of the drug in question have been inserted and distributed that the identification and thus the distribution of another drug shall be allowed. The opening 10 is controlled by the computer 6 and it does not open after the tablets required to meet the existent medical prescription have been introduced.

Preferably, the introduction element 9 may be oriented to allow the operator to find the most comfortable work position in particular also referring to the fact that the operator is right or left handed.

According to a preferred embodiment of the present invention, the introduction element 9 is made up of a hollow tubular arm hinged with an axis vertical to the support structure 2 above the work surface 3 in such a manner to allow the operator to introduce the tablets into the special opening 10 both from the right and from the left.

Suitable transport means transfer the tablets of each drug introduced into the opening 10 of the introduction element 9 up to the obtainment of the personal blisters of the patients corresponding to those the drug in question is intended for.

More in detail, the transport means comprise an intermediate storage element 13, which is susceptible to slidingly move along the longitudinal extension Y of the counter 1 in a track or guide 27 fixed against the support structure 2, for distributing the tablets to the various personal blisters of the patients.

The intermediate storage element 13 is mounted on a moveable carriage 14 bearing - fixed at the lower part - a toothed belt 15 loop wound between two pulleys arranged at the longitudinal ends of the counter one of which is the return pulley and while the other is rotated by a motor controlled by an encoder to accurately displace the carriage 14 along the abovementioned longitudinal direction Y. The intermediate storage element is obtained by means of a rotating turntable 28 rotatingly mounted on the carriage 14 along a vertical axis Z. The turntable peripherally bears a plurality of containers 17, and it is rotated around the axis Z by a stepping motor 18 to separately receive, in the same containers 17, the tablets from the introduction element 9, and for releasing - into the blister - after the movement of the carriage 14 along the longitudinal direction Y.

More in detail, the tablets are inserted one by one into the opening 10 of the introduction element 9 and, through a transfer pipe 9', they exit from an outlet mouth 10' which releases them separately into the containers 17.

For such purpose, the latter are rotated by the turntable by a step upon the arrival of each tablet to separately load the containers each with a tablet up to the overall number of tablets or until all container containers 17 are filled.

Upon filling or after the overall number of tablets for the drug in question has been attained, the intermediate storage element 13 shall be moved longitudinally for distribution into the blister. Should the number of prescribed tablets exceed the capacity of the intermediate storage element the latter shall load and distribute them at different travels.

The carriage 14 displaces the storage element 13 longitudinally along the abovementioned longitudinal direction Y between a loading position, in which the containers 17 separately receive the tablets from the outlet mouth 10' of the transfer pipe 9' of the introduction element 9, and a plurality of release positions, uniquely associated to various personal blisters, in which the containers 17 distribute the tablets to the respective personal blisters through the pipes 11.

For such purpose, the transport means comprise a plurality of fixed transfer pipes 11, each provided with a first end 22 susceptible to receive a tablet at a time from a container 17 of the intermediate storage element 13 when the latter is arranged thereabove, and a second end 12 associated to a corresponding personal blister for unloading the tablet thereinto. The displacement of the carriage 14 and the turntable must be quite accurate in that it must allow the containers 17 to stop at positions indicating the patients and i.e. above the first ends 22 of the transfer pipes 11 corresponding to the selected personal blisters. Arranged at such positions are transparent luminous cards 23 bearing the patient's number and data, such cards being lighted by a special projector 50 mounted in the carriage 14. Therefore, the operator may check, even visually, whether the patient indicated on the monitor is actually the one identified by the intermediate storage element 13.

Provided for is a first plug 21 actuable between a closed position and an opening position when the container 17 is moved by the stepping motor 18 above the same first plug 21 in a dispensing position above the first end 22 of a corresponding pipe 11 to release the tablet into the respective personal blister.

Advantageously, the plug 21 is obtained by moving the moveable bottom whose bottom is engaged with a lever 20 to an actuator when it is positioned in the abovementioned distribution position. More clearly, the turntable rotates to move one of the containers 17 with the tablet to be released up to the preset distribution position in which the bottom of the container 17 intended to release the tablet is positioned coupled to the actuator 19 which first controls the opening to release the tablet and then the closure. Subsequently, once the carriage 14 is displaced in another release position, the turntable rotates to move another container 17 in the distribution position where the actuator 19 may once again control the opening of the bottom 21.

The actuator 19 is controlled by the processor 6 to withhold the drug in one of the containers 17 while the later is not positioned above the opening associated to the first end 22 of the transfer pipe 11, corresponding to the personal blister of the patient for whom the drug is intended.

The second end 12 of each transfer pipe 11 mechanically and operatively bears associated a distributor unit with several positions for various times of the day 25, suitable to introduce the drugs into the compartment of the personal blister provided for by the operative sequence.

The tablet, once released following the opening of the first plug 21, reaches the distributor unit with various positions for various times of the day 25, where a special detector 26 signals the occurred passage thereof into the second end 12 of the transfer pipe and introduction thereof into the personal blister.

Once the drug reaches the personal blister, signalled by the passage detector 26, it allows the processor 6 to move on to the next tablet controlling the displacement of the intermediate storage element 13 with a new container 17 above the first end 22 of the pipe 11 corresponding to the subsequent patient for whom the drug was prescribed.

Once the single drug is inserted into the introduction element 9, a second plug 24 closes the opening 10 so as to prevent the insertion of a new drug before the previous one reaches its destination in the respective container of the intermediate storage element 13. Advantageously, provided for may be a red standby led that switches to green once the drug arrives in the intermediate storage element indicating the opening of the second plug 24 arranged to intercept the introduction element 9 and thus the possibility to introduce a new tablet.

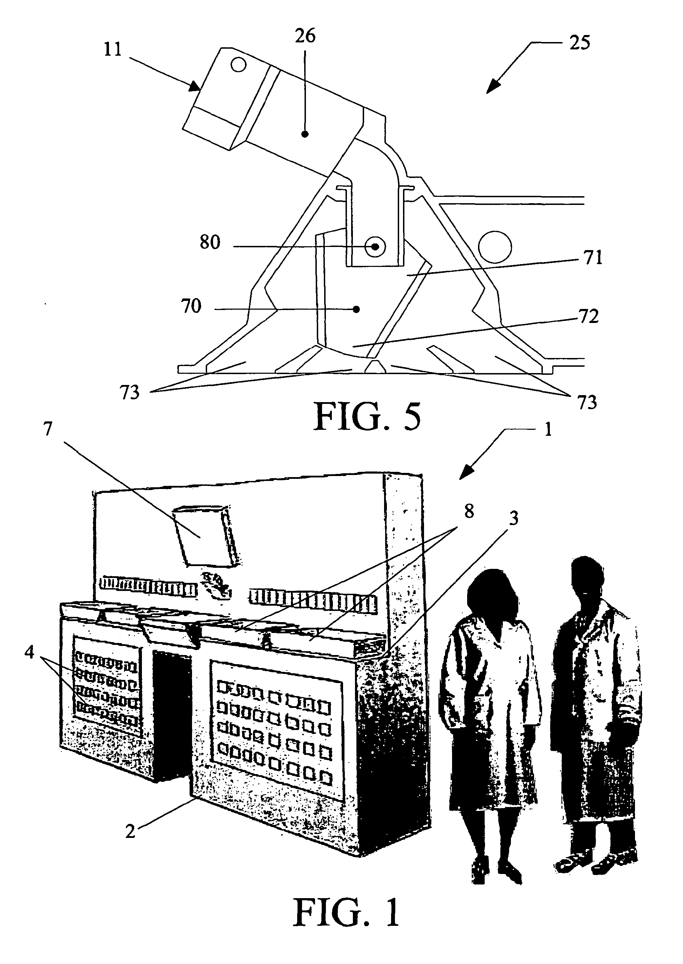

As observable in figure 5 the distributing unit with positions for various times of the day 25 is provided with a diverter 70 made up of a pipe provided with an upper end 71, suitable to receive the tablet from the second end 12 of the transfer pipe 11, and a lower end 72 selectively associable to different discharge channels 73 aimed at directing the tablet into different compartments of the blister. For such purpose, the diverter 70 is hinged into the distributor unit 25, and it is displaceable by means of an actuator, to rotate around a shaft 80 between different angular positions suitable to direct the tablet towards the different discharge channels 73 and thus towards the different compartments of the blister. The actuator - not illustrated in detail in the attached figures in that per se obtainable through different kinematics known to a man skilled in the art, and all falling within the scope of protection of the present patent - simultaneously controls, upon command from the computer system, the diverter 70 of all the distributor units 25, so that - with an angular movement - it is oriented to direct the tablet towards the compartment of the blister provided for its arrival.

The intermediate storage element of the mechanised type described above, contains and electronically manages arrival and release tablet by tablet, and it has several functions. A first function is that of allowing storage of identical tablets, in an extremely quick manner in that it does not require the displacement of the carriage 14 but only a slight angular rotation of the rotating turntable 28. A second function is that of allowing an optimised distribution, and i.e. in progressive succession, of the destination of the stored tablets reducing the distribution time and the number of movements of the electromechanical system. In addition, all this allows operator some time, while the counter executes the distribution task, for the subsequent picking of packages of the drug from the container and the operation of extracting the tablets required for the subsequent introductions from the packaging.

More clearly, the counter 1 mainly described from a structural point of view up to now is suitable to operate as outlined hereinafter.

An operator picks - from one of the containers 8 on the right side - a package of drugs and reads it using the recognition unit 5. Consequently, the monitor 7 displays the name of the drug and the numbered list of the guests that require such drug according to their therapy record. Evidently, the monitor displays the overall number of tablets required for the drug in question in such a manner to allow the operator to possibly go to the storage unit to fetch the remaining amount. Thus, the operator inserts the tablets of the identified drug into the introduction element 9 until the required number is obtained, breaking the packaging blister wearing gloves. The intermediate storage element 13 at the loading position up to this point, upon filling by rotating - step by step - all containers thereof or only those required, moves - driven by the carriage 14 - to distribute the tablets reaching the various release positions according to an optimized special sequence. The release of the tablets requires rotating the turntable having a loaded container 17 at the distribution position where the bottom is opened by the actuator. The position of the intermediate storage element 13 is followed by the operator by means of the lighting of the cards 23 of the patients suitable to indicate the proper positioning of a container 17 to unload the tablet into the pipe 11 of the blister corresponding to the patient for whom the drug in question was prescribed.

Once the tablet reaches the personal blister suitably directed by the distribution means into the compartment indicated by the operative sequence, a further lighting indicator (preferably a flash light overlapped to the previously present and static lighting of the card) and possibly also an acoustic signal indicate the completion of the operation of unloading the drug and the processor moves on to the subsequent step moving the carriage 14 having a container - still loaded - at the first end of the pipe 11 of the blister corresponding to another patient to whom the drug in question was prescribed.

The movements of the intermediate storage element 13 thus continue up to the completion of the operations for distributing the tablets regarding the drug in question as indicated in the therapy record of each guest.

Therefore, in order to introduce the group of identical tablets provided for by the computer system for the drug in question, the operator may monitor the attainment, automatic, of the various unloading positions and see and hear the confirmation of successful release for the subsequent blisters in an optimized sequence.

Upon the tablets in the containers of the intermediate storage element are finished, the carriage 14 returns to the loading position to complete the distribution of tablets of the drug in question or for loading it with another drug.

In the latter case, the operator picks a second type of drug and reads the barcode using the recognition unit 5 and goes ahead, as done previously, up to the completion of the requirements for such new drug.

Thus the operator repeats these operations for all the packages of drugs held in the containers 8. Thus the operator may be deemed to have completed the task thereof if all possible drugs - in the containers 8 - indicated in the therapy records had been taken into account. Otherwise, the computer 6 displays the drugs to be distributed still required to complete the operation of filling the personal blisters.

The computer 6 shall thus allow - through a special printer 40 - printing for example: the list of drugs unloaded in the personal blisters; the list of the generated loaded personal blisters, bearing the name of the guest and the list of drugs administered on that day divided into compartments. The function of electronic loading for computerised management of the storage element shall also allow - for example - producing printouts regarding: requests for drugs according to amounts calculated according to consumption (minimum stock level calculation) and thus aimed at storing a low stock of drugs; lists of packages of drugs approaching the expiry date etc.

All hygienic and handling requirements are met when loading the drugs. For such purpose, the single drug is picked from the packages only when inserting into the introduction element with the aim of avoiding exposure to possibly contaminated environments. Inside the counter, the drugs shall however be deemed to be in protected environments given that the pipes are made of teflon material or a resin suitable for handling drugs and an environment having air that is filtered and treated using vermicide lamps having ultraviolet rays and slightly overpressure.

The transport means 11 provide for conveying the tablet inserted into the opening 10 in the introduction element 9 up to the personal blister corresponding to the patient indicated in the monitor 7, through the distributor unit with several positions for various times of the day 25.

Advantageously, the counter subject of the present invention allows a limited number of types of drugs and may be considered a repackaging means.

The invention thus conceived attains the preset objects.

Obviously the invention may even acquire in the practical embodiment, shapes and configurations different from those illustrated above without departing from the present scope of protection. Furthermore, all details may be replaced by technically equivalent elements and the shapes, dimensions and materials used may vary depending on the requirements.

## Claims

1. Counter for dispensing drugs in form of tablets in personal blisters, comprising:
- a framework (2) positionable against the ground, provided with an operating station;
- a plurality of container cells (4), supported by said framework (2), each of which being susceptible to accommodating in a movable manner at least one personal blister uniquely associable to one patient,
- a packaging recognition unit (5) for identifying drugs in form of tablets held therein;
- an electronic processor (6) provided with a monitor (7), in communication with said recognition unit (5), provided with a data base in which information regarding drugs and the relative recorded prescriptions can be stored per single patients; said processor (6) being adapted to calculate per each drug identified by said recognition unit (5), the overall number of tablets to be dispensed to patients to whom it has been prescribed;
- an introduction element (9) arranged in proximity to said operating station, provided with at least one opening (10) singularly inserted into which are the drugs in tablets;
- transport means for transferring the tablets received by said introduction element (9) into the personal blisters to the patients to whom the corresponding drug has been prescribed, said transport means comprising at least one intermediate storage element (13), which is susceptible to receiving from said introduction element (9) the tablets of a said identified drug, singularly dispensed in a plurality of container vessels (17), said intermediate storage element (13) being actuatable to move between a loading position in which it receives - in its vessels (17) from said introduction element (9) the tablets of said drug, and a plurality of distribution positions, in which it releases the single tablets, contained in said vessels (17), into the personal blisters of the patients to whom they have been prescribed.

2. Counter according to claim 1, **characterised in that** said transport means comprise a plurality of fixed transfer pipes (11), each provided with a first end (22) susceptible to receiving said drugs from a vessel (17) of said intermediate storage element (13) arranged in said distribution position, and with a second end (12) associated to a corresponding personal blister.

3. Counter according to claim 2, **characterised in that** said intermediate storage element (13) comprises a rotating turntable (28) peripherally bearing supports for said container vessels (17), and susceptible to rotating, with said intermediate storage element (13) in one of said distribution positions, to move one of said vessels (17) to the first end (22) of a corresponding pipe (11) to release the tablet into the relative personal blister.

4. Counter according to claim 3, **characterised in that** said vessels (17) are moved, by rotating the turntable (28), above a first plug (21) actuatable between a closure position and an opening position when said vessel (17) is in said distribution position above the first end (22) of a corresponding pipe (11) to release the tablet into the relative personal blister.

5. Counter according to claim 4, **characterised in that** said plug (21) is obtained by means of a moveable bottom usually arranged in closure to withhold the tablet received from said introduction element (9) in said vessel (17), and actuable by means of actuators in opening to release said tablet into said transfer pipe (11).

6. Counter according to claim 1, **characterised in that** said introduction element (9) is provided with a second plug (24) actuable in opening to allow the introduction of a drug, and in closure to prevent the introduction of a drug tablet.

7. Counter according to claim 2, **characterised in that** the second end (12) of each said transfer pipes (11) moves - mechanically and operatively associated - a distributor unit with a plurality of day positions (25) adapted to introduce the drugs into the desired compartment of the personal blister as provided for by said prescription.

8. Counter according to claim 1, **characterised in that** each personal blister is insertable solely into the corresponding cell container (4) provided for being an electronic and/or mechanical introduction identification key.

9. Counter according to claim 1, **characterised in that** it comprises signalling means adapted to indicate the correctness or incorrectness of the personal blisters in the container cells (4).

10. Counter according to claim 1, **characterised in that** said signalling means comprise a passage detector (26) associated to said distributor with a plurality of day positions (25) in such a manner to control the movement of the mechanical parts upon actual passage of drugs and not presumed passage of the same.

## Patentansprüche

1. Schrank zum Verteilen von Medikamenten in Tablettenform in persönliche Medikamentendispenser, der Folgendes umfasst:
- eine Tragkonstruktion (2), die auf den Boden gestellt werden kann und mit einer Arbeitsstation versehen ist;
- eine Vielzahl von Aufnahmezellen (4), die von der Tragkonstruktion (2) getragen werden und von denen jede mindestens einen persönlichen Medikamentendispenser, der einem Patienten in eindeutiger Weise zugeordnet werden kann, so beherbergen kann, dass er wieder herausgenommen werden kann;
- eine Einheit (5) zum Erkennen der Verpackungen, um die darin enthaltenen tablettenförmigen Medikamente zu identifizieren;
- einen mit einem Monitor (7) versehenen elektronischen Rechner (6), der mit der Erkennungseinheit (5) kommuniziert und mit einer Datenbank versehen ist, in der die Informationen über die Medikamente und die zugehörigen für die einzelnen Patienten aufgezeichneten Verschreibungen archiviert werden können; wobei dieser Rechner (6) imstande ist, für jedes von der Erkennungseinheit (5) erkannte Medikament die Gesamtzahl der Tabletten zu berechnen, die an die Patienten auszugeben ist, denen es verschrieben wurde;
- eine Einführeinrichtung (9), die in der Nähe der Arbeitsstation angeordnet ist und über mindestens eine Öffnung (10) zum einzelnen Einführen der tablettenförmigen Medikamente verfügt;
- Fördermittel zum Transferieren der von der Einführeinrichtung (9) empfangenen Tabletten in die persönlichen Medikamentendispenser der Patienten, denen das entsprechende Medikament verschrieben wurde, wobei die Fördermittel mindestens ein Zwischenmagazin (13) umfassen, das von der Einführeinrichtung (9) die Tabletten eines identifizierten Medikaments einzeln in eine Vielzahl von Aufnahmegefäßen (17) verteilt aufnehmen kann, wobei dieses Zwischenmagazin (13) betätigt werden kann, um sich zwischen einer Füllstellung, in der es die Tabletten des Medikaments von der Einführeinrichtung (9) in seinen Gefäßen (17) empfängt, und einer Vielzahl von Verteilstellungen zu bewegen, in denen es die in den Gefäßen (17) enthaltenen einzelnen Tabletten in die persönlichen Medikamentendispenser der Patienten abgibt, denen sie verschrieben wurden.

2. Schrank nach Anspruch 1, **dadurch gekennzeichnet, dass** die Fördermittel eine Vielzahl von ortsfesten Transferkanälen (11) umfassen, von denen jeder mit einem ersten Ende (22), das die Medikamente von einem Gefäß (17) des in der Verteilstellung angeordneten Zwischenmagazins (13) empfangen kann, und einem zweiten Ende (12) versehen ist, das einem entsprechenden persönlichen Medikamentendispenser zugeordnet ist.

3. Schrank nach Anspruch 2, **dadurch gekennzeichnet, dass** das Zwischenmagazin (13) ein Drehkarussell (28) umfasst, das die auf seinen Umfang montierten Aufnahmegefäße (17) trägt und sich mit dem Zwischenmagazin (13) in eine der Verteilstellungen drehen kann, um die Gefäße (17) auf Höhe des ersten Endes (22) eines entsprechenden Kanals (11) zu bringen, um die Tablette in den jeweiligen persönlichen Medikamentendispenser abzugeben.

4. Schrank nach Anspruch 3, **dadurch gekennzeichnet, dass** die Gefäße (17) durch die Drehung des Karussells (28) über einen ersten Verschluss (21) gebracht werden, der zwischen einer Schließstellung und einer Öffnungsstellung betätigt werden kann, wenn sich das Gefäß (17) in der Verteilstellung über dem ersten Ende (22) eines entsprechenden Kanals (11) befindet, um die Tablette in den jeweiligen persönlichen Medikamentendispenser abzugeben.

5. Schrank nach Anspruch 4, **dadurch gekennzeichnet, dass** der Verschluss (21) mit einem beweglichen Boden ausgeführt ist, der normalerweise geschlossen ist, um die von der Einführeinrichtung (9) empfangene Tablette in dem Gefäß (17) zurückzuhalten, und der mit Stellantriebsmitteln zum Öffnen betätigt werden kann, um die Tablette an den Transferkanal (11) abzugeben.

6. Schrank nach Anspruch 1, **dadurch gekennzeichnet, dass** die Einführeinrichtung (9) mit einem zweiten Verschluss (24) versehen ist, der zum Öffnen betätigt werden kann, um die Eingabe eines Medikaments zu ermöglichen, und der zum Schließen betätigt werden kann, um das Eingeben einer Medikamenttablette zu verhindern.

7. Schrank nach Anspruch 2, **dadurch gekennzeichnet, dass** das zweite Ende (12) von jedem Transferkanal (11) mechanisch und operativ verbunden eine Verteileinheit (25) mit mehreren Tagespositionen trägt, die die Medikamente in das gewünschte Fach des persönlichen Medikamentendispensers eingeben kann, das die Verschreibung vorsieht.

8. Schrank nach Anspruch 1, **dadurch gekennzeichnet, dass** jeder persönliche Medikamentendispenser einzig in die entsprechende Aufnahmezelle (4) eingegeben werden kann, da ein elektronischer und/oder mechanischer Kennschlüssel für die Eingabe vorgesehen ist.

9. Schrank nach Anspruch 1, **dadurch gekennzeichnet, dass** er Anzeigemittel umfasst, die anzeigen können, ob die persönlichen Medikamentendispenser richtig oder falsch in die Aufnahmezellen (4) eingegeben wurden.

10. Schrank nach Anspruch 1, **dadurch gekennzeichnet, dass** die Anzeigemittel einen Durchgangsmelder (26) umfassen, der mit der Verteileinheit (25) mit mehreren Tagespositionen verbunden ist, um die Bewegungen der mechanischen Teile bei tatsächlich erfolgten und nicht nur mutmaßlichen Durchgängen zu beeinflussen.

## Revendications

1. Comptoir pour la distribution de médicaments sous forme de comprimés dans des blisters personnels, lequel comprend :
- une structure portante (2) pouvant être posée sur le sol, munie d'une station opérationnelle ;
- une pluralité de cellules de confinement (4), supportées par ladite structure portante (2), chacune desquelles étant susceptible de loger de manière amovible au moins un blister personnel associable de façon univoque à un patient,
- une unité de reconnaissance (5) des conditionnements pour identifier les médicaments en comprimés contenus dans ceux-ci ;
- un processeur électronique (6) muni d'un écran d'affichage (7), en communication avec ladite unité de reconnaissance (5), muni d'une base de données dans laquelle peuvent être archivées des informations sur les médicaments et sur les ordonnances correspondantes enregistrées pour les patients individuels ; ledit processeur (6) étant adapté pour calculer pour chaque médicament identifié par ladite unité de reconnaissance (5), le nombre total de comprimés à distribuer aux patients pour lesquels il est prescrit ;
- un introducteur (9) disposé à proximité de ladite station opérationnelle, muni d'au moins une ouverture (10) dans laquelle introduire individuellement les médicaments en comprimés ;
- des moyens de transport pour transférer les comprimés reçus en provenance dudit introducteur (9) dans les blisters personnels des patients qui ont en ordonnance le médicament correspondant, lesdits moyens de transport comprenant au moins un magasin intermédiaire (13), lequel est susceptible de recevoir en provenance dudit introducteur (9) les comprimés d'un dit médicament identifié, distribués individuellement dans une pluralité de récipients de confinement (17), ledit magasin intermédiaire (13) étant actionnable pour se déplacer entre une position de chargement dans laquelle il reçoit dans ses récipients (17), en provenance dudit introducteur (9), les comprimés dudit médicament, et une pluralité de positions de distribution, dans lesquelles il délivre les comprimés individuels, contenus dans lesdits récipients (17), dans les blisters personnels des patients qui les ont en ordonnance.

2. Comptoir selon la revendication 1, **caractérisé en ce que** lesdits moyens de transport comprennent une pluralité de conduits de transfert fixes (11), chacun muni d'une première extrémité (22) susceptible de recevoir lesdits médicaments provenant d'un récipient (17) dudit magasin intermédiaire (13) placé dans ladite position de distribution, et d'une deuxième extrémité (12) associée à un blister personnel correspondant.

3. Comptoir selon la revendication 2, **caractérisé en ce que** ledit magasin intermédiaire (13) comprend un carrousel tournant (28) portant lesdits récipients de confinement (17) montés de manière périphérique, et susceptible de tourner, avec ledit magasin intermédiaire (13) dans une desdites positions de distribution, pour amener un desdits récipients (17) en correspondance de la première extrémité (22) d'un conduit correspondant (11) pour délivrer le comprimé dans le blister personnel correspondant.

4. Comptoir selon la revendication 3, **caractérisé en ce que** lesdits récipients (17) sont amenés, par le biais de la rotation du carrousel (28), au-dessus d'un premier obturateur (21) actionnable entre une position de fermeture et une position d'ouverture quand ledit récipient (17) est dans ladite position de distribution au-dessus de la première extrémité (22) d'un conduit correspondant (11) pour délivrer le comprimé dans le blister personnel correspondant.

5. Comptoir selon la revendication 4, **caractérisé en ce que** ledit obturateur (21) est obtenu au moyen d'un fond mobile normalement disposé en fermeture pour retenir dans ledit récipient (17) le comprimé reçu en provenance dudit introducteur (9), et actionnable avec des moyens actionneurs en ouverture pour délivrer ledit comprimé audit conduit de transfert (11).

6. Comptoir selon la revendication 1, **caractérisé en ce que** ledit introducteur (9) est muni d'un deuxième obturateur (24) actionnable en ouverture pour permettre l'introduction d'un médicament et en fermeture pour empêcher l'introduction d'un comprimé de médicament.

7. Comptoir selon la revendication 2, **caractérisé en ce que** la deuxième extrémité (12) de chaque dit conduit de transfert (11) porte, associée mécaniquement et fonctionnellement, une unité distributrice à plusieurs positions de journée (25) adaptée pour introduire les médicaments dans le compartiment désiré du blister personnel prévu par ladite ordonnance.

8. Comptoir selon la revendication 1, **caractérisé en ce que** chaque blister personnel est insérable uniquement dans la cellule de confinement correspondante (4), une clé de reconnaissance électronique et/ou mécanique pour l'insertion étant prédisposée.

9. Comptoir selon la revendication 1, **caractérisé en ce qu'**il comprend des moyens de signalisation adaptés pour indiquer le fait que l'introduction des blisters personnels dans le cellules de confinement (4) est correcte ou non.

10. Comptoir selon la revendication 1, **caractérisé en ce que** lesdits moyens de signalisation comprennent un détecteur de passage (26) associé à ladite unité distributrice à plusieurs positions de journée (25) dans le but de conditionner les mouvements des parties mécaniques pour des passages de comprimés survenus et non supposés.
